## Europäisches Patentamt

## European Patent Office

## Office européen des brevets

(19)

(11) Veröffentlichungsnummer: **0 258 789**
**B1**

(12) **EUROPÄISCHE PATENTSCHRIFT**

(45) Veröffentlichungstag der Patentschrift:
07.02.90

(21) Anmeldenummer: 87112316.2

(22) Anmeldetag: 25.08.87

(51) Int. Cl.⁴: **C07D 303/27**

(54) Verfahren zur Herstellung von 2,2-Di- [p-glycidoxi-cyclohexyl]- propan.

(30) Priorität: 30.08.86 DE 3629632

(43) Veröffentlichungstag der Anmeldung:
09.03.88 Patentblatt 88/10

(45) Bekanntmachung des Hinweises auf die Patenterteilung:
07.02.90 Patentblatt 90/6

(84) Benannte Vertragsstaaten:
BE CH DE FR GB LI NL

(56) Entgegenhaltungen:
DE-A- 2 132 547
US-A- 2 943 095

CHEMICAL ABSTRACTS, Band 82, Nr. 16, 21. April 1975,
Seite 52, Zusammenfassung Nr. 99079k, Columbus,
Ohio, US; & JP-A-74 20 173 (MITSUBISHI CHEMICAL
INDUSTRIES CO., LTD) 07-09-1970

(73) Patentinhaber: BASF Aktiengesellschaft,
Carl-Bosch-Strasse 38, D-6700 Ludwigshafen(DE)

(72) Erfinder: Schuster, Ludwig, Dr., Weinheimer Strasse 44,
D-6703 Limburgerhof(DE)

ACTORUM AG

**Beschreibung**

Die katalytische Hydrierung des Oxiranringes verläuft sehr leicht. So wird beispielsweise bei der Hydrierung von 1,2-Epoxi-1-Phenyl-buten das 1-Phenylbutanol erhalten (D. Ahragam U. Y. Deux, C. r. 205, 285 β1937'). In gleicher Weise wird bei der Hydrierung von Epoxiverbindungen, die daneben einen aromatischen Kern tragen, ganz allgemein stets nur der Oxiranring unter Alkoholbildung aufgespalten; der Benzolring bleibt unangegriffen (Houben Weyl, Band 6/3, S. 445).

Es wurde nun gefunden, daß man 2,2-Di-[p-glycidoxi-cyclohexyl]-propan durch katalytischen Hydrierung in direkt weiterverarbeitbarer Form erhält, wenn man 2,2-Di-[p-glycidoxi-phenyl]-propan in Gegenwart eines Rutheniumkatalysators bei Temperaturen im Bereich von 20 - 60°C und Drücken oberhalb von 100 bar hydriert.

Es ist überraschend, daß bei der katalytischen Hydrierung des Bisglycidylethers des 2,2-Dioxidiphenylpropans, des Bisphenol-A, an Rutheniumkatalysatoren abweichend von dem bekannten Schema nicht die Oxiranringe, sondern die beiden aromatischen Ringe zu der entsprechenden Di-cyclohexanverbindung abgesättigt werden.

Als Katalysator kann man Ruthenium entweder auf inerten Trägern, wie Aktivkohle oder, bevorzugt, auf dem Oxidhydrat, das durch Fällung aus einer wäßrigen Lösung von Rutheniumtrichlorid gewonnen worden ist, für die Hydrierung einsetzen.

Die Menge des verwendeten Hydrierkatalysators ist in geringem Maße abhängig von der Reinheit des Ausgangsproduktes. Im allgemeinen soll die Katalysatorkonzentration im Hydriergemisch zwischen ein und drei Gewichtsteilen Ruthenium pro 1000 Gewichtsteile Bisglycidylether liegen.

Es ist zweckmäßig, nach beendeter Reaktion den Edelmetallkatalysator abzutrennen und erneut einzusetzen. Sollte dies infolge einer Vergiftung durch Verunreinigungen des Ausgangsproduktes, wozu in erster Linie Chlor - entweder organisch gebunden oder als Chlorid - gehört, nicht mehr möglich sein, so empfielt sich eine Aufarbeitung nach bekannten Methoden, beispielsweise über das Rutheniumtetroxid.

Die Hydrierung wird bei Drücken oberhalb 100 bar durchgeführt; im allgemeinen werden Drücke von 200 bis 325 bar angewendet.

Es ist zweckmäßig, die Hydrierung in Lösung durchzuführen. Als Lösungsmittel werden bevorzugt Ether verwendet wie Methyl-tert.-butylether oder Glykoldimethylether; insbesondere haben sich Tetrahydrofuran und Dioxan als vorteilhaft erwiesen. Es kann noch in sehr konzentrierten Lösungen gearbeitet werden, beispielsweise in einer 55-prozentigen Lösung in Tetrahydrofuran.

Bei der Auswahl der Reaktionstemperatur sind verhältnismäßig enge Grenzen gesetzt. So werden bei höheren Temperaturen die Oxiranringe geöffnet, bei zu niedrigen Temperaturen hingegen die aromatischen Systeme noch nicht abgesättigt. Der optimale Temperaturbereich für die selektive Kernhydrierung liegt zwischen 20 und 60°C, bevorzugt zwischen 40°C und 50°C.

Das 2,2-Di-[p-glycidoxi-cyclohexyl]-propan ist ein Ausgangsstoff oder Bestandteil von Epoxidharzsystemen.

Beispiel

In einem mit einem Magnetrührer versehenen Autoklav von 3,5-l-Inhalt werden 1,2 kg Bisphenoldiglycidylether in 1,1 l Tetrahydrofuran gelöst. Zu dieser Lösung werden 10 g einer Rutheniumoxidhydratpaste mit einem Gehalt von 1,4 g Ruthenium hinzugegeben. Die Hydrierung wird unter einem Druck von 300 bar bei einer Temperatur von 50°C ausgeführt. Die gesamte Wasserstoffaufnahme dauert 10 Stunden.

Der Hydrieraustrag wird über Bleicherde und Aktivkohle abfiltriert und eingedampft.

Das so erhaltene Rohprodukt kann direkt weiterverarbeitet werden. Ein besonders reines und helles Produkt erhält man durch Destillation im Vakuum. Unter einen Druck von 0,1 mb geht die Verbindung zwischen 160°C und 180°C über.

**Patentansprüche**

1. Verfahren zur Herstellung von 2,2-Di-[p-glycidoxicyclohexyl]-propan durch katalytische Hydrierung, dadurch gekennzeichnet, daß man 2,2-Di-[p-glycidoxi-phenyl]-propan in Gegenwart eines Rutheniumkatalysators bei Temperaturen im Bereich von 20 - 60°C und Drücken oberhalb von 100 bar hydriert.

**Claims**

1. A process for the preparation of 2,2-di-[p-glycidyloxycyclohexyl]-propane by catalytic hydrogenation, wherein 2,2-di-[p-glycidyloxyphenyl]-propane is hydrogenated in the presence of a ruthenium catalyst at 20–60°C an under a pressure above 100 bar.

**Revendications**

1. Procédé de préparation de 2,2-di-(p-glycidyloxycyclohexyl)-propane par hydrogénation catalytique, caractérisé en ce qu'on hydrogène du 2,2-di-(p-glycidoxy-phényl)-propane en présence d'un catalyseur à base de ruthénium à des températures dans la gamme de 20 – 60° C et sous des pressions de plus de 100 bars.